# EUROPEAN PATENT APPLICATION

(11) **EP 3 936 043 A1**
(43) Date of publication of application: **12.01.2022**
(21) Application number: 20184764.7
(22) Date of filing: 08.07.2020
(51) Int. Cl.: A61B 5/08, A61B 5/01, A61B 5/107, A61B 5/00, A61B 1/233

(54) **SYSTEMS AND METHODS SYSTEM FOR ASSESSING NASAL PATHOLOGIES OF A SUBJECT**

(71) Applicant: Koninklijke Philips N.V., 5656 AG Eindhoven (NL)
(72) Inventor: HIWALE, Sujitkumar Sureshrao, 5656 AE Eindhoven (NL); RADHAKRISHNAN, Ravindranath, 5656 AE Eindhoven (NL); JOHNSON, Mark Thomas, 5656 AE Eindhoven (NL); BERA, Deep, 5656 AE Eindhoven (NL)
(74) Representative: Philips Intellectual Property & Standards

(57) **Abstract**

The invention provides a system for assessing nasal pathologies of a subject. The system includes a processor configured to receive a plurality of proximity signals representative of a respective plurality of detected distances of an inner surface of a nostril of the subject from one or more proximity sensors. The processor processes the plurality of proximity signals to determine one or more characteristics of the plurality of detected distances. Based on the determined one or more characteristics of the detected distances, an indication of one or more nasal pathologies is determined.

## Description

### FIELD OF THE INVENTION

The invention relates to the field of clinical nasal examination, and more specifically to investigation of nasal pathologies.

### BACKGROUND OF THE INVENTION

The investigation and monitoring of nasal health is field undergoing continuous development. Pathologies of the nasal cavities, nasal septum and nostrils are often important cause for a visit to a doctor. Pathologies of ear include: deviated nasal septum; perforation in the nasal septum; nasal polyp; hard crust; turbinal hypertrophy; and nosebleed (epistaxis). For a primary care physician, it is important to properly assess these pathologies, and either treat or refer them to respective specialists.

Currently, nasal investigations often involve physical examination of the nasal cavity using a nasal speculum (anterior rhinoscopy). Nasal speculum is an instrument used to widen the opening of a nostril so the inside can be more easily seen. These methods are widely used, but are time consuming and the quality of the results is heavily dependent upon the skill of the operator. Moreover, other mitigating factors such as presence of turbinate hypertrophy, rhinitis, nasal valve collapse, nasal cycle and the complexity of the three dimensional geometry of the nasal cavity make the diagnosis nasal pathologies even more challenging.

Apart from speculum examination, endoscopy, acoustic rhinometry (AR), rhinomanometry (RMM), nasal spectral sound analysis (NSSA), multislice CT and MR are other methods, which can be used to assess nasal pathologies. Acoustic rhinometry (AR) assesses nasal patency based on the measurement of acoustic reflection of a sound signal in the nose by structures within the nasal cavity. Rhinomanometry provides a dynamic physiologic assessment of the nose by measuring transnasal pressure and nasal volume airflow to calculate nasal resistance. Nasal sound spectral analysis can provide an indirect method of dynamically assessing nasal airflow by analyzing noise in the nasal cavity caused by turbulent nasal airflow. As such, no simple method exists for quick detection of common nasal pathologies.

There is therefore a need for a simple means of reliable nasal investigation that does not depend on the skill of the operator.

### SUMMARY OF THE INVENTION

The invention is defined by the claims.

According to examples in accordance with an aspect of the invention, there is provided a system for assessing nasal pathologies of a subject, the system comprising:
a processor configured to:
receive a plurality of proximity signals representative of a respective plurality of detected distances of an inner surface of a nostril of the subject from one or more proximity sensors;
process the plurality of proximity signals to determine one or more characteristics of the plurality of detected distances; and
determine an indication of one or more nasal pathologies based on the determined one or more characteristics of the detected distances.

It is proposed that detected distances of an inner surface of a nostril of a subject from one or more proximity sensors may be leveraged and analysed to obtain an understanding of the shape and/or volume of the subject's nasal cavity. Based on this, an indication of one or more nasal pathologies may be determined. By way of example, the inventors have realized that a probe with a proximity sensing arrangement may be inserted into a nostril of a subject and provide distance measurements, thus enabling the shape and/or characteristics of the subject's nostril(s) to be determined. By processing information relating to detected distances in various way, nasal pathologies may be quickly and easily inferred. Thus, it is proposed that a nasal probe or nasal speculum provided with proximity sensors can be used to provide information about the characteristics of a subject's nasal cavity, and this information may be processed in accordance with various proposed methods to provide an indication of one or more nasal pathologies.

Thus, unlike conventional investigation approaches which rely on the skilled application of a speculum (or other medical equipment) by a trained and/or experienced medical professional, proposed embodiments may not need to rely on complicated and/or subjective visual inspection techniques. Instead, it is proposed to leverage distance information obtained by simple insertion of a probe into a subject's nostril(s).

For instance, it is proposed that embodiments may be employed in conjunction with a nasal probe or nasal speculum that has integrated proximity sensors (such as time-of-flight sensors) which, when inserted into a one or both nostrils of a subject, detect distances of an inner surface of a nostril of the subject from the proximity sensor(s) (e.g. compute the distance based on the time-of-flight). Such a nasal probe may comprise a single arm (i.e. probe or shaft) for insertion into one nostril of a subject at a time, or may alternatively comprise two arms for insertion into both nostrils of subject at the same time.

The length of the arm(s) may be adjustable, so at to enable insertion into the nostril(s) to varying depths. Similarly, a distance between two arms may be adapted to adjustable according to requirements. The tips of both the arms are embedded with the proximity sensors. Shafts of both the arms are embedded with an array of proximity sensors in multiple directions (at least four directions perpendicular to each other). A tip of an arm may be inserted in each nostril and will be slowly moved inside the nostril. The arm may be kept in the middle of the nostril or can be moved up-down, left-right or in a circular manner inside the nostril to cover all the areas

Proximity sensors may be used to probe a nasal cavity at various depths to detect an anatomical structure and measure its distance along the length of the nasal cavity. During an examination process, a nasal probe may be inserted in each nostril independently/sequentially (or two nasal probes may be inserted simultaneously into both nostril) and detected distance values may then be analysed to identify anatomical landmarks/features. Accordingly, not only may a nasal patholgy be identified using the acquired distance information, but embodiment may also determine the position of the nasal pathology in the nostril(s).

In this way, the system may be adapted to interpret the signals obtained by way of the proximity sensors provided on a nasal probe to determine an indication of nasal pathologies present in the nostril(s) of a subject. By way of example, one or more nasal pathologies may comprise at least one of: deviated nasal septum; perforation in the nasal septum; nasal polyp; hard crust; turbinal hypertrophy; and nosebleed.

In an embodiment, the processor may be further configured to determine at least one property of the one or more nasal pathologies based on the determined one or more characteristics of the detected distances, and preferably wherein the at least one property comprises one more of: a size; a shape; a location; a depth; and a measure of severity.

The plurality of proximity signals may be representative of a respective plurality of detected distances with respect to the same nostril of the subject,

One or more characteristics of the plurality of detected distances may comprise one or more of: variation in distance values; deviation from an expected distance value; comparison against a maximum threshold value; and comparison against a minimum threshold value. By considering a variety of different characteristics of the detected distances, it will be appreciated that the accuracy and flexibility of the system for arriving at a determination of the indication of a nasal pathology may be improved. For example, by considering a variety of signal features, the most prominent features of a given signal may be selected in order to obtain an accurate comparison, which may then be used to obtain an accurate indication of a nasal pathology.

The processor may be configured to determine an indication of deviated nasal septum based on the determined variation in distance values and/or the determined deviation from an expected distance values. For instance, a gradual increase/decrease in detected distance value with insertion depth may indicate the gentle toward/away bend (i.e. deviation) in the nasal septum.

Simiiarly, the processor may be configured to determine an indication of perforation in the nasal septum based on the determined variation in distance values and/or the determined deviation from an expected distance values For instance, a sudden/step increase in detected distance value with insertion depth may indicate the presence of a hole (i.e. perforation) in the nasal septum.

By way of further example, the processor may be configured to determine an indication of a perforation of the nasal septum of the subject based on the determined comparison against a maximum threshold value, and preferably wherein the maximum threshold value is based on a depth of the one or more proximity sensors in the nostril of the subject. The maximum threshold value may representative of a maximum expected value of detected distance to an inner surface of a nostril (i.e. expected width of a nostril). If a deteced distance exceeds this maximum expected value, it may be indicative of an inner surface not be present (i.e. a hole or perforation in the septum).

Embodiments may further comprise a nasal probe comprising one or more proximity sensors adapted to be inserted into a single nostril of the subject, wherein each of the one or more proximity sensors is adapted to detect, when inserted into the nostril of the subject, a respective distance of an inner surface of the nostril to the proximity sensor, and to generate a respective proximity signal representative of the detected distance. Thus, embodiments may employ a nasal probe that is adapted to be inserted into a single nostril of a subject at once. When inserted, the proximity sensor(s) may generated proximity signals representative of detected distances of the proximity sensor(s) to an inner surface of the nostril. The proximity signals may thus provide information about the size, shape and/or internal volume of the nostril, and this information may be processed in accordance with proposed concepts to determine an indication of one or more nasal pathologies.

In some embodiments, the plurality of proximity signals may be representative of a respective plurality of detected distances with respect to different nostrils of the subject. Further, one or more characteristics of the plurality of detected distances may comprise one or more of: difference in distance values; and comparison against a predetermined value. Also, the processor may be configured to determine an indication of at least one of: a thickness of nasal septum; and a deviated nasal septum based on the determined difference in distance values. For instance, a gradual increase in detected distance with insertion depth may be indicatve of the septum deviating away from the proximity sensor(s).

The processor may be configured to determine an indication of a perforation in the nasal septum of the subject based on the determined difference in distance values and/or the determined comparison against a predetermined value. Also, the predetermined value may based on a depth of the one or more proximity sensors in the nostril of the subject.

Some embodiments may further comprise a nasal probe comprising first and second arms adapted to be simultaneously inserted into the first and second nostrils of the subject, respectively, wherein each of the first and second arms comprises at least one of the one or more proximity sensors, each proximity sensor being adapted to detect, when inserted into a nostril of the subject, a respective distance of an inner surface of the nostril to the proximity sensor, and to generate a respective proximity signal representative of the detected distance. Thus, embodiments may employ a nasal probe that is adapted to be inserted into both nostrils of a subject at once.

Also, the processor may be configured to determine an indication of a perforation in the nasal septum of the subject based on the determined comparison against a predetermined value. For example, the predetermined value may be substantially equal to the distance between a proximity sensor on the first arm and a proximity sensor of the second arm. The predetermined value may thus representative of a distance between arms of the nasal probe. If a deteced distance equals this value, it may be indicative of an inner surface not be present between the arms of the probe (i.e. a hole or perforation in the septum).

Some embodiments may further comprise: a nasal probe comprising first and second arms adapted to be simultaneously inserted into the first and second nostrils of the subject, respectively, wherein the first arm comprises a transmitter adapted to transmit an optical signal and wherein the second arm comprises an optical detector adapted to detect an optical signal transmitted by the transmitter and to generate a detection signal responsive to detect an optical signal transmitted by the transmitter, and wherein the processor is configured to receive a generated detection signal from nasal probe and to determine an indication of a perforation in the nasal septum of the subject based on the received detection signal. Put another way, if a receiver one arm opposite a tranimtter on the other arm receives a signal from the transmitter, it may be indicative of an inner nostril surface not be present between the two arms of the probe (i.e. a hole or perforation in the septum).

The processor may be configured to: identify respiratory cycles of the subject; and determine an indication of a nasal polyp based on a temporal variation in the plurality of detected distances during the identified respiratory cycles. Further, the processor may be configured to identify respiratory cycles of the subject based on at least one of: temperature signals representative of a detected temperature values in a nostril of the subject; and a temporal variation in the plurality of detected distances. Accordingly, various different supplementary sensors (e.g. thermometer, camera, accelerometer, gyroscope, air flow sensor, etc.) may be employed to verify the distance measurement and correlate those with physical changes or events occurring in the nose of a subject, so as to identify respiration of the subject for example.

The processor may be further configured to: determine a guidance indication of based on the determined one or more characteristics of the detected distances. In this way, embodiments may use the detected distances to guide a user for correct insertion and positioning of the nasal probe and/or promity sensors within a subject's nostril(s). Embodiments may thus facilitate nasal investigation by untrained and/or unsupervised users.

In some embodiment, determining an indication of one or more nasal pathologies is further based on analyzing the proximity signals with a machine learning algorithm, wherein the machine learning algorithm has been trained to output an indication of nasal pathologies. By using a machine-learning algorithm, such as one of the variety of known machine learning algorithms, the accuracy of the determination of the indication of the nasal pathology may be improved. Further, the determination of the indication of the nasal pathology may be performed with reduced input data.

According to another aspect of the invention, there is provided a computer implemented method for assessing nasal pathologies of a subject, the method comprising:
receiving a plurality of proximity signals representative of a respective plurality of detected distances of an inner surface of a nostril of the subject from one or more proximity sensors;
processing the plurality of proximity signals to determine one or more characteristics of the plurality of detected distances; and
determining an indication of one or more nasal pathologies based on the determined one or more characteristics of the detected distances.

According to examples in accordance with an aspect of the invention, there is provided a computer program product comprising computer program code means which, when executed on a computing device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a schematic representation of a system for assessing nasal pathologies of a subject according to an embodiment;
Figure 2 depicts a method for calibration of a two-armed probe according to an embodiment;
Figure 3 shows a method for assisting navigation of a two-armed probe inside a subject's nasal cavity according to an embodiment;
Figure 4 shows a method for assisting alignment of a two-armed probe during insertion into a subject's nasal cavity according to an embodiment; and
Figure 5 is a schematic illustration of a two-armed nasal probe being used to identify perforation of a nasal septum according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for assessing nasal pathologies of a subject. The system comprises a processor. The processor is configured to receive a plurality of proximity signals representative of a respective plurality of detected distances of an inner surface of a nostril of the subject from one or more proximity sensors. The processor is also configured to process the plurality of proximity signals to determine one or more characteristics of the plurality of detected distances. The processor is yet further configured to determine an indication of one or more nasal pathologies based on the determined one or more characteristics of the detected distances.

The term indication may refer to the presence or absence of a given nasal pathology.

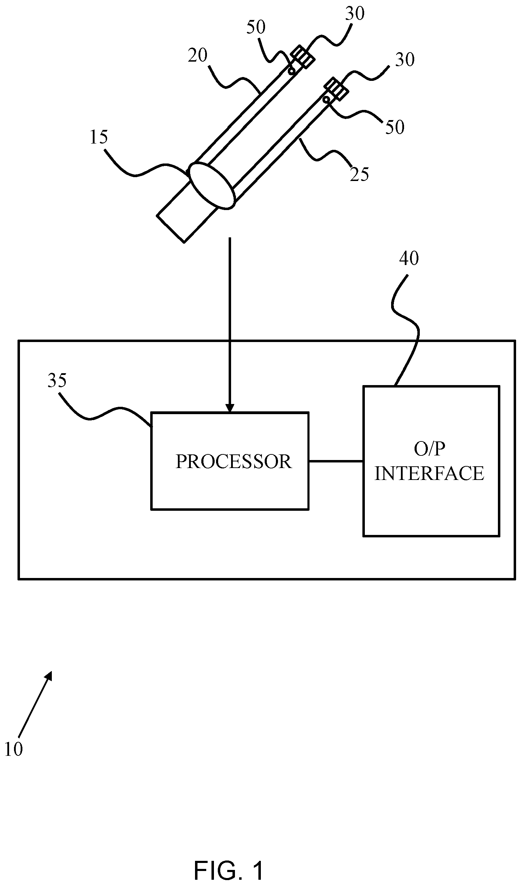

Figure 1 shows a system 20 for assessing nasal pathologies of a subject according to an embodiment.

In particular, Figure 1 shows a nasal probe 15 comprising first 20 and second 25 arms adapted to be simultaneously inserted into the first and second nostrils of the subject, respectively. Each of the first 20 and second 25 arms comprises at least one or more proximity sensors 30, each proximity sensor being adapted to detect, when inserted into a nostril of the subject, a respective distance of an inner surface of the nostril to the proximity sensor, and to generate a respective proximity signal representative of the detected distance. By way of example, the proximity sensors may be time-of-flight sensors (e.g. Laser or IR).

The system comprises a processor 35 and an output (O/P) interface 40 (which in this example comprises a display unit 40).

The processor 35 is configured to receive, from the nasal probe 15, a plurality of proximity signals representative of a respective plurality of detected distances of an inner surface of a nostril of the subject from the one or more proximity sensors. The processor 35 is also configured to process the received proximity signals to determine one or more characteristics of the plurality of detected distances. Based on the determined one or more characteristics of the detected distances, the processor 35 determines an indication of one or more nasal pathologies.

For example, one or more characteristics of the plurality of detected distances may comprise one or more of: variation in distance values; deviation from an expected distance value; comparison against a maximum threshold value; and comparison against a minimum threshold value. For instance, the processor 35 may be configured to determine an indication of a perforation in the nasal septum of the subject based on the determined comparison against a predetermined value. For example, the predetermined value may be substantially equal to the distance between a proximity sensor on the first arm and a proximity sensor of the second arm. The predetermined value may thus representative of a distance between arms of the nasal probe. If a deteced distance equals this value, it may be indicative of an inner surface not be present between the arms of the probe (i.e. a hole or perforation in the septum).

The processor 35 provides the indication of one or more nasal pathologies to the O/P interface 40 for output to a user of the system 10 (by way of visual signal provided by the O/P interface 40).

In this exemplary embodiment, the processor 35 is also configured to determine at least one property of the one or more nasal pathologies based on the determined one or more characteristics of the detected distances. For instance, the at least one property comprises one more of: a size; a shape; a location; a depth; and a measure of severity.

It is also noted that, in this embodiment, each arm 20, 25 also comprises a respective temperature sensor 50 (e.g. thermister). Each temperature sensor 50 is adapted to generate, when inserted into a nostril of a subject, temperature signals representative of detected temperature values in the nostril of the subject. Accordingly, the processor 35 is adapted to receive the generated temperature signals and to identify respiratory cycles of the subject based on the temperature signals and temporal variations in the plurality of detected distances. In this way, temperature signals may be employed to verify the detected distances and/or correlate those with physical changes or events occurring in the nose of a subject, so as to identify respiration of the subject for example.

From the above-described example, it will apprecaiated that proposed systems and methods are based on acquiring proximity signals representative of detected distances of an inner surface of a nostril of the subject from one or more proximity sensors. These distances may be used to obtain information about the shape and/or size of the nostrils and thus identify features that may be indicative of nasal pathologies.

Main elements of an exemplary embodiment may comprise:
(i) an array of Time-of-flight (i.e. proximity) sensors provided on the shaft and/or tip of a nasal probe;
(ii) a processor adapted to collect data from the sensors and determine an indication of one or more nasal pathologies based on the collected data from the time-of-flight sensors.

When the nasal probe is inserted into the nostril(s) of a subject, the proximity sensors are activated sequentially to find the distance to the nearest structure. The distance data can be used to profile the nasal cavity. For example, the data can be used to identify if the septum is deviated or not. Also, information from a temperature sensor can be used to measure the change in temperature in the nasal cavity while inspiring and expiring. Such information about temperature change can be used to identify the respiration rate.

Embodiments may also be adapted to provide guidance to user of the nasal probe, so as to ensure correct insertion, alignment and orientation in the nostril(s) of the subject. For instance, the nasal probe may be provided with an accelerometer and gyroscope arrangement that is configured to monitor and orientation of the probes. Based on signals from the accelerometer and gyroscope arrangement, the may determine a guidance indication of th user. In this way, embodiments guide a user to correctly insert and position of the nasal probe and/or promity sensors within a subject's nostril(s). This may facilitate nasal investigation by untrained and/or unsupervised users.

Determining an indication of nasal pathologies may be performed by analyzing the proximity signals with a machine-learning algorithm, wherein the machine learning algorithm has been trained to output an indication of a nasal pathology.

A machine-learning algorithm is any self-training algorithm that processes input data in order to produce or predict output data. Here, the input data may comprise: the characteristics of the characteristics of the detected distances; subject data, such as subject age, subject gender; a known subject condition; subject demographic data; and a diagnosis of the subject. The output data may comprise an indication of a nasal pathology.

Suitable machine-learning algorithms for use in embodiments will be apparent to the skilled person. Purely by way of example, suitable machine-learning algorithms may include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

According to an example embodiment, the training input data entries correspond to example characteristics of detected distances, and the training output data entries correspond to an example indication of a nasal pathology.

It will be appreciated from the above description that proposed embodiments process proximity signals to determine one or more characteristics of detected distances, and then determine an indication of one or more nasal pathologies based on the determined one or more characteristics of the detected distances. Examples of determining an indication of various nasal pathologies will therefore now be described with reference to proposed concepts.

### - Deviation of Nasal Septum (DNS) determined using a single-armed nasal probe

Deviation of the nasal septum is a common cause of structural nasal obstruction. Data from a single-armed nasal probe provided with proximity sensors may be used to determine an indication of DNS using an exemplary approach as follows:
(a) As a simple handheld portable device, a digital nasal probe with only one arm/prong can be used for a quick detection of presence or absence of DNS.
(b) The probe is inserted into the middle of one of the subject's nostrils and is slowly moved deeper inside the nostril (generally parallel to the nasal septum). The nasal probe comprises a single Time-of-flight sensor on the inner side (i.e. facing nasal septum) preferably near the tip (i.e. distal end) of the probe. Alternatively, multiple sensors can be integrated on the inner side.
(c) The Time-of-flight sensor continuously measures a distance of the nearest object (which in the nasal septum in this case) on the inner side as the speculum is moved inside the nasal cavity.
(d) The Nasal septum is a midline structure, which separates two nostrils. Therefore, it should be equidistance from the Time-of-flight sensor of the nasal speculum throughout the length of the nasal cavity.
(e) The inner distance measurements from the Time-of-flight sensor throughout the length of the nasal cavity are analyzed to find any significant change in the distance along the course of the nasal speculum. For example, a proposed algorithm may use the difference between two subsequent distance measurements throughout the length of the nasal cavity to determine presence/absence of any DNS and its direction.
(f) For example, if the distance of a nearest object (Di) detected by the Time-of-flight sensor on the inner side of the nasal cavity is less than the previous nearest object (Di-1) by a predetermined tolerance value (e.g., 2 mm), an embodiment may determine an indication that the septum is bent towards the senor along the path. This may be indicative that nasal septum is deviated towards the side of sensor. The tolerance value may be proximity sensor properties or empirical values.
(g) On the other hand, if Di is greater than Di-1 by the predetermined tolerance value, an embodiment may determine an indication that the septum is bent towards the opposite side (creating a concavity towards the senor along the path), which results in increase in distance between sensor and the nasal septum. This may be indicative that nasal septum is deviated away from the side of sensor.

### - Perforation of Nasal Septum determined using a single-armed nasal probe

Nasal septum perforations are breach in continuity of nasal speculum. A perforated septum can vary in size and location, and are usually found deep inside the nose. Data from a single-armed nasal probe provided with proximity sensors may be used to determine an indication of a nasal septum perforation using an exemplary approach as follows:
(a) A digital nasal speculum with only one prong can be used for quick detection of a nasal septal perforation.
(b) The probe is inserted into the middle of one of the subject's nostrils and is slowly moved deeper inside the nostril (generally parallel to the nasal septum). The nasal probe comprises a single Time-of-flight sensor on the inner side (i.e. facing nasal septum) preferably near the tip (i.e. distal end) of the probe. Alternatively, multiple sensors can be integrated on the inner side.
(c) The Time-of-flight sensor continuously measures a distance of the nearest object (which in the nasal septum in this case) on the inner side as the speculum is moved inside the nasal cavity.
(d) The inner distance measurements from the Time-of-flight sensor throughout the length of the nasal cavity are analyzed to find any significant change in the distance along the course of the nasal speculum. For example, a proposed algorithm may use the difference between two subsequent distance measurements throughout the length of the nasal cavity to determine presence of a nasal septum perforation.
(e) In general, the nasal cavity is widest at its anterior part near the floor and measures about 1.5 cm. Therefore, it is expected that the nasal septum would be within this limit from the sensor. Thus, if the detected distance from the proximity sensors to nasal septum exceeds this distance (i.e. a maximum threshold value), then it indicates breach in continuity of the nasal septum. The threshold value for identifying the presence of a nasal perforation may, for example, be determined based attributes/properties of the subject, such as age, gender, race and/or specific values of nasal cavity dimension.
(f) In a case of extreme deviation of nasal septum on other side, it is possible to have septum placed (at its deepest concave point) beyond the Perforation threshold when measured from the midline. However, as septum deviates gently it is less likely to bend beyond the Perforation threshold abruptly within a very short distance. Therefore, if the distance between two consecutive measurements by the sensor (i.e. the distance between Dᵢ and Dᵢ₋₁) is small, then septum bending would be within the 'Perforation threshold'.

The above examples have been described with reference to employing the use of a single-armed probe. However, it should be appreciated that proposed concepts are also applicable to the use of two-armed (i.e. dual-pronged) nasal probes that are adapted to be inserted into both nostrils of a subject at the same time. As a simple example, a two-armed nasal probe may be similar to that depicted in Figure 1, wherein a set of proximity sensors is provided near the tip (i.e. distal end) of each arm. The proximity sensors are preferably configured to point (at least) towards the opposite arm, so as to be directed towards the nasal septum when the two-armed nasal probe in inserted into the nostrils of a subject).

When such a two-armed probe is employed, some embodiments may also include concepts of activation and/or calibration of the probes (for example to ensure correct orientation or alignment of the probe).

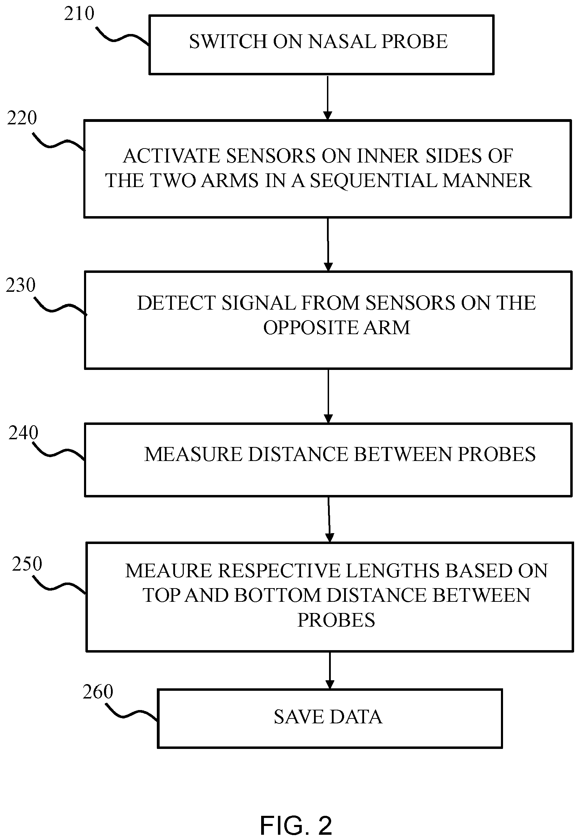

Purely by way of example, a method for calibration of a two-armed probe according to an embodiment is depicted in Figure 2.

The nasal speculum is switched on (i.e. activated) 210, and the method then proceed to activating 220 the top and bottom most proximity sensors of each prong/arm (on inner side) are activated in a sequential manner to detect distance of two prongs from each other. Signals from the sensors on the opposite arm are detected (step 230) and then the distance between the probes are measured (step 240) based on the detected signals from the sensors of the other arm. The measure distance between probes at top and bottom is used to determine if the two probes are parallel to each other or not. Also, in step 250, the distance between probes for the topmost (i.e. closest to the distal end) and bottommost (i.e. closest to the proximal end) is also used to determine respective lengths of the two probes.

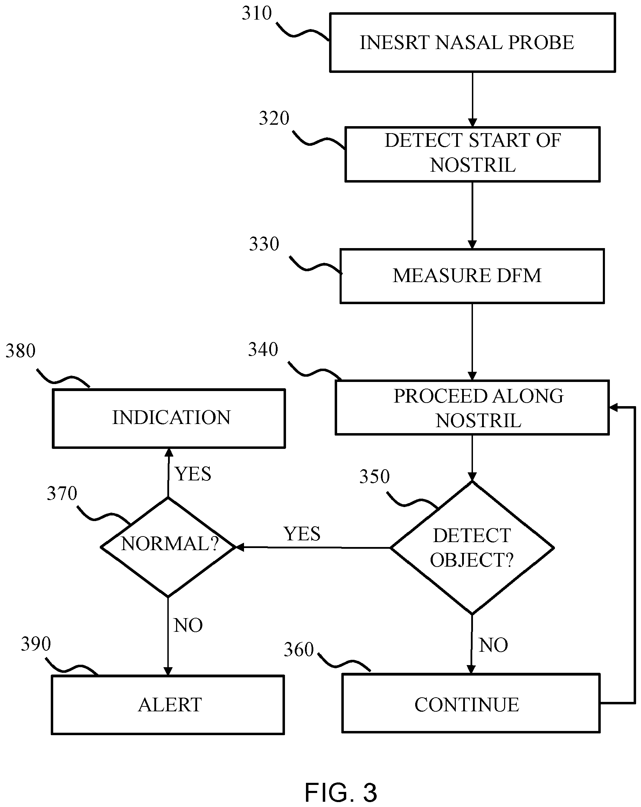

Purely by way of further example, a proposed method for assisting navigation of a two-armed probe inside a subject's nasal cavity is depicted in Figure 3.

The nasal probe is firstly inserted into both of the subject's nostrils (step 310). When the arms of the nasal probe enter the nostrils, the proximity sensors on the inner side of each arm identify start of medial wall of the nostril; this detects the start of respective nasal cavities (step 320).

The distance of each arm from the start of the medial wall of the nostril is measured (step 330) and this is considered as the distance of the individual arms from the midline (otherwise referred to as the distance from the midline (DFM)).

The probe is inserted further into the nostrils of the subject (step 340). The proximity sensors at the tip of each prong are used to guide the continued insertion on each arm inside the respective nostrils. If, in step 350, no object or potential obstacle to insertion is detected, the user is prompted (step 360) to continue insertion of the probe into the nostrils and the method returns to step 340. However, if in step 350, an object or potential obstacle to insertion is detected, the detected object/obstacle is assessed (step 370) to determine if it is within normal/expected anatomical limits. If the detected object/obstacle is not within normal/expected anatomical limits, and alert is communicated to the user (step 390). Conversely, if the detected object/obstacle is within normal/expected anatomical limits, an indication is provided to the user (step 380). Thus, when the proximity sensors of the probe detect an obstacle, the user may be provided with an audio-visual-haptic indication. In this way, the user can be assisted in inserting the nasal probe into the nasal cavity without touching/hurting any internal structures.

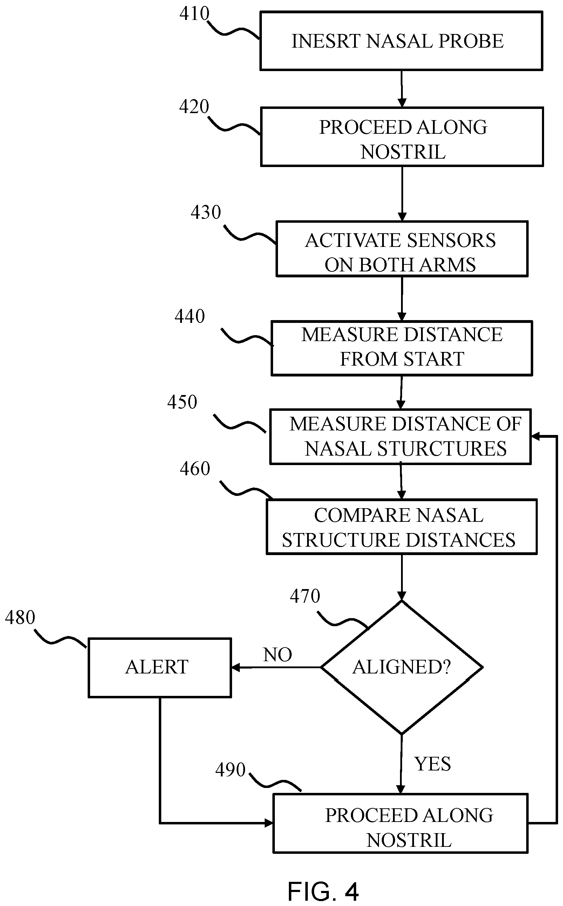

Yet further, a proposed method for assisting alignment of a two-armed probe during insertion into a subject's nasal cavity is depicted in Figure 4.

The two-armed probe is firstly inserted into the subject's nostrils (step 410) and then along the nostrils to a greater depth (step 420) at which time the proximity sensors of the probe are activated (step 430). The proximity sensors are used to probe the nasal cavity at various depths to measure distance of nasal structures in all four directions along the length of each nostril (steps 440 and 450). In doing so, each arm of the probe may be kept in the middle of the nostril or can be moved up-down, left-right or in a circular manner inside the nostril to cover all the areas.

The nasal septum is a midline structure, which separates two nostrils. When nasal septum is in exactly center it will be equidistance from the both arms of the probe (when they are inserted in the two nostrils at the same distance from the midline). The distance of the two probes on inner sides at the starting of nasal cavity (DFM) can thus continuously measured to check if the two probes are at equal distance from the midline.

Accordingly, the distances between the two probes and floor of the nasal cavity are measured (step 460) and then compared (step 470) to check whether the two arms of the probe are aligned properly with respect to each other or not. If it is determined based on the measure distances that the arms are not properly aligned, an alert is communicated to the user of the probe (step 480). Conversely, if it is determined based on the measure distances that the arms are properly aligned, an indication of proper alignment and/or instruction to proceed with continued insertion of nasal probe is communicated to the user (step 490) and the method then returns to continued measuring of the distances (step 450).

Alternatively, accelerometer and/or gyroscope sensors (or similar) may be used to monitor rotational angle around the axis of the probes to determine alignment of the arms of a two-armed nasal probe.

### - Perforation of Nasal Septum determined using a two-armed nasal probe

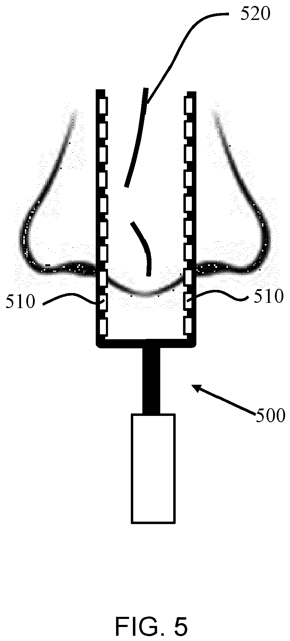

With reference to the illustration of Figure 5, data from a two-armed nasal probe 500 provided with proximity sensors 510 (facing each other on opposite arms) may be used to determine an indication of a nasal septum 520 perforation using an exemplary approach as follows:
(a) To detect septal perforation, the proximity sensors 510 on the inner side of both probe arms are activated may be sequentially one after other (with the proximity sensors of one arm acting as transmitter, and the proximity sensors of the other arm acting as receiver).
(b) When there is no perforation in the nasal septum 520, there should be no cross-talk between the two arms (i.e. reception at one arm of signals transmitted from the other arm). Therefore, if a sensor of an arm receives any signal from the sensor of the other arm, this indicates a perforation in the nasal septum.
(c)If there is a perforation in the nasal septum 520, a proximity sensor of one arm may detect the opposing proximity sensor of the other arm as the nearest object (assuming they are properly aligned). This detected distance will be equal to the distance between the arms.
(d) Information about detected distances of surfaces from the proximity sensors 510 can be used to detect presence of nasal septum perforation. Exemplary distance measurements obtained using a two-armed nasal probe are provided in Table 1 and Table 2 below.

**Table 1**

| Distance from nasal opening (i.e.insertion depth) (mm) | Right nostril: outer side | Right nostril: up | Right nostril: inner side | Right nostril: down | Left nostril: outer side | Left nostril: up | Left nostril: inner side | Left nostril: down |
|---|---|---|---|---|---|---|---|---|
| 5 | 8 | 35 | 7 | 10 | 8 | 35 | 7 | 10 |
| 7 | 9 | 34 | 7 | 11 | 9 | 34 | 7 | 11 |
| 9 | 6 | 35 | **18** | 10 | 6 | 36 | **18** | 10 |
| 11 | 5 | 34 | **18** | 11 | 5 | 34 | **18** | 11 |
| 13 | 4 | 36 | 7 | 10 | 4 | 37 | 7 | 10 |

From the Table 1, it can be observed that probes on both the sides have detected the nearest objects at the same distance, which is equal to the distance between the arms of the nasal probe. This indicates that there is nasal septum perforation at depths of 9 to 11 mm.

A case of misaligned probes is illustrated in Table 2 below, wherein the probe arm thickness is assumed to be 4 mm.

**Table 2**

| Distance from nasal opening (mm) | Right nostril: outer side | Right nostril: up | Right nostril: inner side | Right nostril: down | Left nostril: outer side | Left nostril: up | Left nostril: inner side | Left nostril: down |
|---|---|---|---|---|---|---|---|---|
| 5 | 8 | 35 | 7 | 10 | 8 | 35 | 7 | 10 |
| 7 | 9 | 34 | 7 | 11 | 9 | 34 | 7 | 11 |
| 9 | 6 | 35 | **28** | 12 | 6 | 36 | **28** | 10 |
| 11 | 5 | 34 | **28** | 13 | 5 | 34 | **28** | 11 |
| 13 | 4 | 36 | 7 | 14 | 4 | 37 | 7 | 10 |

From the Table 2, it can be observed that two probes have difference in their down sensors reading, which indicates misalignment. It is can be observed that right the side has detected the nearest obj ects at the distance of 28 mm, which is equal to the distance between arms of the nasal probe plus outer side distance at the same depth (6 mm) plus probe arm thickness (4 mm). This indicates that there is nasal septum perforation at depths of 9 to 11 mm.

The information obtained from these readings can be also used to quantify size of nasal septum perforation.

### - Measurement of Nasal Septum deviation and thickness using a two-armed nasal probe

Data from a two-armed nasal probe provided with proximity sensors may be used to determine an indication of a nasal septum deviation using an exemplary approach as follows:
(a) The proximity sensors on the arms of the nasal probe are used to probe the nasal cavity at various depths to measure distance of nasal structures in all four directions along the length of each nostril. Example distance measurements obtained in this manner are provided in Table 3 below. The information from the two nostrils can be correlated to identify anatomical landmarks in the nasal cavity and to determine position of the nasal septum in relation to the two nostrils.

**Table 3**

| Distance from nasal opening (mm) | Right nostril: outer side | Right nostril: up | Right nostril: inner side | Right nostril: down | Left nostril: outer side | Left nostril: up | Left nostril: inner side | Left nostril: down |
|---|---|---|---|---|---|---|---|---|
| 0 = DFM | | | **7** | | | | **7** | |
| 5 | 8 | 35 | **7** | 10 | 8 | 35 | **7** | 10 |
| 7 | 9 | 34 | **6** | 11 | 9 | 34 | **8** | 11 |
| 9 | 6 | 35 | **2** | 10 | 6 | 36 | **13** | 10 |
| 11 | 5 | 34 | **1** | 11 | 5 | 34 | **13** | 11 |
| 13 | 4 | 36 | **3** | 10 | 4 | 37 | **11** | 10 |

For instance, from the example measurement of Table 3 above, it can be observed that two nostrils have different dimensions on the inner sides. This indicates that the nasal septum is closer to the right side probe at depths of 9 to 13 mm. The distance measurements from the left side indicate that the nasal septum is away from the probe at depths of 9 to 13 mm. This indicates that the nasal septum is deviated towards the right side.

### -Quantifying DNS Bending

Data from a two-armed nasal probe provided with proximity sensors may also be used to quantify DNS bending. For example, the degree of DNS bending for each side can be calculated as follows, Bending = (Right nostril: inner side - Right nostril: DFM)

Example distance measurements obtained from a two-armed nasal probe in this are provided in Table 4 below.

**Table 4**

| Distance from nasal opening | Right nostril: inner side | Bending on Right (mm) | Left nostril: inner side | Bending on left (mm) |
|---|---|---|---|---|
| 0 = DFM | 7 | | 7 | |
| 5 | 7 | 0 | 7 | -1 |
| 7 | 6 | 1 | 8 | -6 |
| 9 | 2 | 5 | 13 | -6 |
| 11 | 1 | 6 | 13 | -4 |
| 13 | 3 | 4 | 11 | -1 |

From the Table 4 above, it can be observed the nasal septum is deviated towards the right side by maximum of 6 mm at the depth of 11 mm.

### -Quantifying Nasal Septum Thickness

Data from a two-armed nasal probe provided with proximity sensors may also be used to determine nasal septum thickness. In particular, the distance between two probe arms (DBP) can be used to calculate the thickness of nasal septum at various length of the nasal cavity as follows: Septum thickness = DBP- (distance of septum on right + distance of septum on left).

For example, if distance between two probe arms (DBP) is 16 mm, the nasal septum thickness at various depths for the observations in Table-5 below can be calculated.

**Table 5**

| Distance from nasal opening | Right nostril: inner side | Left nostril: inner side | Septum thickness (mm) |
|---|---|---|---|
| 0 = DFM | 7 | 7 | **2** |
| 5 | 7 | 7 | **2** |
| 7 | 6 | 8 | **2** |
| 9 | 2 | 13 | **1** |
| 11 | 1 | 13 | **2** |
| 13 | 3 | 11 | **2** |

From Table 5 above, it can be observed the nasal septum is thinnest at the depth of 9 mm.

The information from the different depths in the two nostrils can be collectively used to quantify degree of nasal septum deviations and its thickens at various depths. Using this, detailed anatomical details of the nasal septum can be provided to a user.

### -Detection of nasal polyp(s) using a nasal probe

Nasal polyps are abnormal growth in the nasal cavity, which can originate from any portion of nasal mucosa or paranasal sinuses. Nasal polyps are usually benign, semitransparent, well defined structures originating from roof of the nasal cavity. They can vary in size from a few millimeters to centimeters. Nasal polyps usually result from chronic inflammatory conditions. Multiple polyps in the nasal cavity can be also found in a few diseases conditions.

Nasal polyps can lead to nasal obstruction, chronic sinusitis, anosmia, headache, snoring, which can eventually hamper quality of life. The clinical manifestation of nasal polyp depends on the size of polyp. Small polyps may not produce any noticeable symptoms and may an accidental finding during nasal examination, if they are located in anterior part of nasal cavity. However, nasal polyps are usually located deep inside the nasal cavity and hence are difficult to diagnose by routine nasal examination.

Proposed embodiment may be used to provide an indication of the presence of one or more nasal polyps as follows:
(i) As has already been described above, a nasal probe comprising one or more proximity sensors is inserted in to the nostril(s) of a subject.
(ii) The proximity sensor(s) are used to probe the nasal cavity at various depths to measure distance of nasal structureslong the length of the nostril(s).
(iii) Any abnormal masses in a nasal cavity may be detected by analyzing the dimension(s) of the nasal cavity at various depth and comparing it with expected dimensions.
(iv) Further, the proximity sensor(s) can be used to detect inspiration and expiration by monitoring movements of walls of nostrils. Here, it is noted that the lateral wall of the nostril moves outward during the expiration whereas it moves inward during inspiration. Alternatively, a temperature sensor provided on the nasal probe may be used to measure changes in temperature within the nasal cavity while inspiring and expiring. Temperature change data can be used to measure the respiration rate and indicate if the patient is inhaling or exhaling. Since a pedunculated nasal polyp typically moves with respiratory cycles (whereas other anatomical structures in the nasal cavity do not move), if there is a detected change in the dimensions on inside of nasal cavity, the presence of a nasal polyp inside the nasal cavity may be identified.

### - Detection of nosebleed in the nasal cavity using a nasal probe

A nasal probe may also be used to detect active bleeding in the nasal cavity by measuring temperature inside the nasal cavity.

The nasal cavity is constantly exposed to atmospheric air. A temperature gradient from anterior to posterior parts of the nasal cavity can therefore be observed (based on outside temperature and humidity).

Temperature measurements obtained from inside the nostrils of a subject at various depths, along with outside temperature and humidity, can be used to generated a matrix of expected temperature at various depths and dimensions of the nasal cavity. A model based on a representative database can be also used to generate such matrix.

The temperature of active bleeding spot is assumed to close to core body temperature (37°c)

Using the above information and expected temperature at various depths, a bleeding spot can be identified inside a nasal cavity.

### - Identification of anatomical structure in the nasal cavity using data from a nasal probe

A nasal speculum with integrated proximity sensing capabilities can be also used to identify various structure in the nasal cavity, e.g. by measuring cross section/dimension of the nasal cavity at various depths and comparing it with reference models of nasal cavity. This information can be used to measure dimension of various internal structures (e.g., turbinate) or to detect their abnormalities. The information can be also used to detect any obstacles/obstructions in the nasal cavity.

### - Determination of nasal cavity volume using a nasal probe

From Table 3 above, it is also possible to determine the approximate volume of the nasal cavities. The nasal cavities can be assumed as having a conical shape with the base at the nostrils. For instance, the radius of the nostrils and the height of the nasal cavities can be determined from the measurements shown in Table 3 above.

It is also possible to extract the diameters at different levels inside the nasal cavities. The volume (V) of the individual nasal cavities may then be computed as V = 1/3 πr² h, where, r = radius of the nostril/at different depth of the nasal cavities and h = the height of the nasal cavity.

Such information might be used to rendered a 3D deformed cone (as the diameter at depths can vary) visualization of the volume for the ease of interpretation.

Difference in the volumes of the left and right nostrils may also indicate potential deviation of the nasal septum or other pathologies.

Whilst the previous embodiments have been described with a set of discrete proximity sensors, in alternative embodiments, the discrete proximity sensors could be replaced or augmented by any (semi-)continuous proximity sensor. Known proximity sensors of this class include 3D laser scanner systems (using a laser beam and a rotating mirror arrangement or similar), which are often used to gain a 3D image of an empty volume. Making use of a miniaturized version of such a sensor at the end of the arm of a nasal probe may enable the immediate build-up of a 3D image of a significant part of the nasal cavity in just a few image capture sessions. Such an image may allow the extraction of clinically relevant parameters in an even more detailed manner.

From the above-described embodiments, it will be understood that it is proposed that detected distances of an inner surface of a nostril from one or more proximity sensors may be analyzed to determine indication of one or more nasal pathologies. Such distances may be obtained from a proximity sensing arrangement that is inserted into the nostril(s) of a subject.

By processing information relating to detected distances in various ways, nasal pathologies may be quickly and easily inferred. Thus, it is proposed that a nasal probe provided with proximity sensors can be used to provide information about the characteristics of a subject's nasal cavity, and this information may be processed in accordance with various proposed methods to provide an indication of one or more nasal pathologies.

Thus, embodiments may be employed in conjunction with a nasal probe that has integrated proximity sensors (such as time-of-flight sensors) which, when inserted into a one or both nostrils of a subject, detect distances of an inner surface of a nostril of the subject from the proximity sensor(s). Such a nasal probe may comprise a single probe (i.e. arm or shaft) for insertion into one nostril of a subject at a time, or may alternatively comprise two probes for simultaneous insertion into both nostrils of subject.

During an examination process, a nasal probe may be inserted in each nostril independently/sequentially (or two nasal probes may be inserted simultaneously into both nostril) and detected distance values may then be analyzed to identify nasal pathologies. By way of example, embodiments may analyze obtained distance measurement to provide an indication of: deviated nasal septum (DNS); perforation in the nasal septum; nasal polyp; hard crust; turbinal hypertrophy; and/or nosebleed.

Proposed embodiments may thus address the problem of identifying and quantifying a DNS, thus avoiding a need for subjective assessment. Embodiments may also address a difficulty associated with locating a DNS when it is deep inside the nasal cavity or has a complex shape with more than one bend/curve.

Proposals may also be used to provide information on the thickness of a subject's nasal septum, which may be important for surgical repair. 4. Embodiments may further provide the advantage of being able to determine a precise location and size of septal perforation.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system for assessing nasal pathologies of a subject, the system comprising:
a processor configured to:
receive a plurality of proximity signals representative of a respective plurality of detected distances of an inner surface of a nostril of the subject from one or more proximity sensors;
process the plurality of proximity signals to determine one or more characteristics of the plurality of detected distances; and
determine an indication of one or more nasal pathologies based on the determined one or more characteristics of the detected distances.

2. The system of claim 1, wherein the one or more nasal pathologies comprise at least one of: deviated nasal septum; perforation in the nasal septum; nasal polyp; hard crust; turbinal hypertrophy; and nosebleed.

3. The system of claim 1 or 2, wherein the processor is further configured to:
determine at least one property of the one or more nasal pathologies based on the determined one or more characteristics of the detected distances, and preferably wherein the at least one property comprises one more of: a size; a shape; a location; a depth; and a measure of severity.

4. The system of any of claims 1 to 3, wherein the plurality of proximity signals are representative of a respective plurality of detected distances with respect to the same nostril of the subject,
and wherein one or more characteristics of the plurality of detected distances comprise one or more of:
variation in distance values;
deviation from an expected distance value;
comparison against a maximum threshold value; and
comparison against a minimum threshold value.

5. The system of claim 4, wherein the processor is configured to determine an indication of deviated nasal septum based on the determined variation in distance values and/or the determined deviation from an expected distance value.

6. The system of claim 4 or 5, wherein the processor is configured to determine an indication of a perforation of the nasal septum of the subject based on the determined comparison against a maximum threshold value, and preferably wherein the maximum threshold value is based on a depth of the one or more proximity sensors in the nostril of the subj ect.

7. The system of any of claims 4 to 6, further comprising:
a nasal probe comprising one or more proximity sensors adapted to be inserted into a single nostril of the subject, wherein each of the one or more proximity sensors is adapted to detect, when inserted into the nostril of the subject, a respective distance of an inner surface of the nostril to the proximity sensor, and to generate a respective proximity signal representative of the detected distance.

8. The system of any of claims 1 to 3, wherein the plurality of proximity signals are representative of a respective plurality of detected distances with respect to different nostrils of the subject,
and wherein one or more characteristics of the plurality of detected distances comprise one or more of:
difference in distance values; and
comparison against a predetermined value,
and preferably wherein the processor is configured to determine an indication of at least one of: a thickness of nasal septum; and a deviated nasal septum based on the determined difference in distance values.

9. The system of claim 8, wherein the processor is configured to determine an indication of a perforation in the nasal septum of the subject based on the determined difference in distance values and/or the determined comparison against a predetermined value, and preferably wherein the predetermined value is based on a depth of the one or more proximity sensors in the nostril of the subject.

10. The system of claim 8 or 9, further comprising:
a nasal probe comprising first and second arms adapted to be simultaneously inserted into the first and second nostrils of the subject, respectively,
wherein each of the first and second arms comprises at least one of the one or more proximity sensors, each proximity sensor being adapted to detect, when inserted into a nostril of the subject, a respective distance of an inner surface of the nostril to the proximity sensor, and to generate a respective proximity signal representative of the detected distance.

11. The system of claim 10, wherein the processor is configured to determine an indication of a perforation in the nasal septum of the subject based on the determined comparison against a predetermined value, and wherein the predetermined value is substantially equal to the distance between a proximity sensor on the first arm and a proximity sensor of the second arm.

12. The system of any of claims 8 to 10, further comprising:
a nasal probe comprising first and second arms adapted to be simultaneously inserted into the first and second nostrils of the subject, respectively,
wherein the first arm comprises a transmitter adapted to transmit an optical signal and wherein the second arm comprises an optical detector adapted to detect an optical signal transmitted by the transmitter and to generate a detection signal responsive to detect an optical signal transmitted by the transmitter,
and wherein the processor is configured to receive a generated detection signal from nasal probe and to determine an indication of a perforation in the nasal septum of the subject based on the received detection signal.

13. The system of any of claims 2 to 12, wherein the processor is configured to:
identify respiratory cycles of the subject; and
determine an indication of a nasal polyp based on a temporal variation in the plurality of detected distances during the identified respiratory cycles,
and preferably wherein the processor is configured to identify respiratory cycles of the subject based on at least one of:
temperature signals representative of a detected temperature values in a nostril of the subject; and
a temporal variation in the plurality of detected distances.

14. The system of any of claims 1 to 13, wherein the processor is further configured to:
determine a guidance indication based on the determined one or more characteristics of the detected distances.

15. A computer implemented method for assessing nasal pathologies of a subject, the method comprising:
receiving a plurality of proximity signals representative of a respective plurality of detected distances of an inner surface of a nostril of the subject from one or more proximity sensors;
processing the plurality of proximity signals to determine one or more characteristics of the plurality of detected distances; and
determining an indication of one or more nasal pathologies based on the determined one or more characteristics of the detected distances.
